# EUROPEAN PATENT APPLICATION

(11) **EP 4 717 224 A1**
(43) Date of publication of application: **01.04.2026**
(21) Application number: 24203261.3
(22) Date of filing: 27.09.2024
(51) Int. Cl.: A61B 34/30, A61B 90/50, A61F 9/009, A61F 9/00

(54) **SURGICAL ROBOTIC SYSTEM COMPRISING EYE MANIPULATION SYSTEM**

(71) Applicant: Carl Zeiss Meditec AG, 07745 Jena (DE)
(72) Inventor: MEENINK, Hildebert Christiaan Matthijs, 5656 AG Eindhoven (NL); AAN HET ROT, Steven, 5656 AG Eindhoven (NL)
(74) Representative: DeltaPatents B.V.

(57) **Abstract**

A surgical robotic system 200 is provided which comprises an eye manipulation system. The eye manipulation system may be configured for orbital manipulation of an eye of a patient. The eye manipulation system may comprise an arm 132 having at least one degree of freedom and an eye gripping device 130 mounted to a distal end of the arm. The eye gripping device may be configured to engage an eyeball 102 by exerting a grip on a fibrous surface layer of the eyeball, and the arm may be adjustable in pose to move the eye gripping device along a curved plane 131 representing a curvature of the eyeball to, when the eyeball is engaged by the eye gripping device, rotate the eyeball within an eye socket 104.

## Description

### TECHNICAL FIELD

The presently disclosed subject matter relates to surgical robotic system for ophthalmic surgery which comprises an eye manipulation system for orbital manipulation of an eye of a patient.

### BACKGROUND

Ophthalmic surgical procedures increasingly involve the use of surgical robotic systems. Rather than operating entirely autonomously, such surgical robotic systems are expected for the near future to remain at least in part under the control of a human operator. For example, the human operator may directly or indirectly control the movement of a surgical instrument mounted to a surgical arm of the surgical robotic system. Nevertheless, it is expected that future surgical robotic systems may be more autonomous and require less or even no involvement of a human operator.

A surgical robotic system designed for ophthalmic surgical procedures may be provided with an instrument manipulator for mounting a surgical instrument. The instrument manipulator may have at least one degree of freedom to enable adjustment of a pose of the surgical instrument. An actuation subsystem may be provided which may be arranged to effect the adjustment of the pose of the surgical instrument. Here, the term 'pose' may refer to a position and orientation of surgical instrument. Through such actuation, the surgical instrument may for example be moved in a lateral direction, for example relative to an anatomical surface such as a retinal surface, and/or in a longitudinal direction along a longitudinal axis of the surgical instrument. Longitudinal movement may typically allow the surgical instrument to be moved towards and away from a surgical target. Accordingly, the surgical instrument may be used to modify (biological) tissue near the surgical target, to deliver an agent to the surgical target, etc. Examples of surgical instruments include, but are not limited to, forceps, mechanical cutters, coagulation cutters, scissors, injection needles, sealing devices, etc.

While the use of surgical robotic systems in ophthalmic surgical procedures offers numerous benefits, including enhanced precision and dexterity, it may be required that the pose of the eye relative to the surgical instrument is known during surgery so that the pose of the surgical instrument may be appropriate controlled. Here, the term `pose of the eye' may include the position and orientation of the eye. The position of the eye may be determined by or based on a position of the head of a patient. The orientation of the eye may be determined by the orientation of the head and by the orientation of the eye within the eye socket. For example, during intraocular surgery, the head of the patient may be brought into a known position and orientation and the orientation of the eye within the eye socket may be fixated. For example, it is known to establish a coupling between the surgical robotic system and the eye by the surgical robotic system using a trocar interface to couple with a trocar inserted into the eye, thereby fixating the orientation of the eye with respect to the surgical robotic system.

Next to the static fixation of the eye, there may also be a need to manipulate the orientation of the eye during an ophthalmic surgical procedure. Such manipulation may involve rotating the eye within the eye socket and thereby changing the orientation of the eye with respect to one or more external surgical entities, such as the instrument manipulator of a surgical robotic system, a microscope, etc. The externally induced change in the orientation of the eye may also be referred to as orbital manipulation since it involves altering the eye's orientation within the orbital cavity. Orbital manipulation may be advantageous for various reasons, including but not limited to improving the visualization or observability of peripheral regions of the eye and enhancing the reach of surgical instruments within the eye.

It is known to manually perform orbital manipulation. For example, a surgeon may rotate the eye using two instruments that are inserted into the eye.

There also exist robotic systems that can perform orbital manipulation of the eye. For example, the paper "Vitreoretinal Surgical Robotic System with Autonomous Orbital Manipulation using Vector-Field Inequalities" by Koyama et al., 2023, https://arxiv.ora/abs/2302.05567, describes autonomous orbital manipulation of the eye in robot-assisted vitreoretinal surgery, in which a surgical needle is used as a first surgical instrument and a light guide as a second surgical instrument to jointly rotate the eye within the eye socket. The paper is concerned with determining a control strategy to appropriately control both surgical instruments to manipulate the eye.

However, there is a need for an improved eye manipulation system, in particular an improved robotic eye manipulation system.

### SUMMARY

An aspect of the presently disclosed subject matter provides a surgical robotic system for ophthalmic surgery, comprising:
- an instrument manipulator for mounting a surgical instrument, the instrument manipulator having at least one degree of freedom to enable adjustment of a pose of the surgical instrument;
- a first actuation subsystem arranged to effect the adjustment of the pose of the surgical instrument;
- an arm having at least one degree of freedom;
- an eye gripping device mounted to a distal end of the arm, wherein the eye gripping device is configured to engage an eyeball by exerting a grip on a fibrous surface layer of the eyeball;
- a second actuation subsystem arranged to effect a movement of the eye gripping device along a curved plane representing a curvature of the eyeball to, when the eyeball is engaged, rotate the eyeball within an eye socket.

The above aspect of the invention involves providing a surgical robotic system which comprises an instrument manipulator for mounting a surgical instrument. Instrument manipulators for surgical robotic systems are known per se in the field of medical robotics, and may take over tasks of a surgeon's hand, for example by handling and manipulating a surgical instrument. A surgical instrument may be a tool or device for performing actions to carry out desired effects during a surgery or operation, such as modifying biological tissue or providing access to observe the biological tissue. Examples of surgical instruments include, but are not limited to, forceps, mechanical cutters, coagulation cutters, scissors, injection needles, sealing devices, etc.

The instrument manipulator may have at least one degree of freedom (DoF) to enable a pose of the surgical instrument to be adjusted. Here, the term 'pose' may refer to the position and/or orientation of the surgical instrument. As such, by way of the at least one DoF of the instrument manipulator, the position and/or orientation of the surgical instrument may be adjusted. To provide the instrument manipulator with the at least one DoF, the instrument manipulator may for example be mounted to a surgical arm. For example, the surgical arm may comprise a linkage system to which the instrument manipulator may be movably suspended, such as a parallelogram linkage mechanism. The linkage system may be connected to a base. It is noted that the instrument manipulator may also be provided with the at least one DoF using other mechanisms besides a surgical arm, such as linear actuators, a gantry system, etc.

The surgical instrument may typically have a longitudinal axis which may typically pass through a tip of the surgical instrument. The at least one DoF may enable the tip of the surgical instrument to be moved, for example laterally in relation to an anatomical surface, such as a retinal surface or the surface of another structure within or on the eye. Typically, the instrument manipulator may be provided with three or more DoFs, for example to enable longitudinal movement of the surgical instrument along the longitudinal axis, for example to advance towards and/or retract away from a surgical target, and to enable lateral movement of the tip of the surgical instrument along a plane perpendicular to the longitudinal axis. It is noted that instrument manipulators having the functionality described in this paragraph and in the preceding paragraph may be known per se from the field of medical robotics.

An actuation subsystem may be provided for effecting the adjustment of the pose of the surgical instrument. For example, the actuation subsystem may comprise one or more actuators to actuate the instrument manipulator and/or to actuate components through which the instrument manipulator is movably suspended, such as the aforementioned surgical arm, such as the parallelogram linkage system, and/or the base. Another term for actuation subsystem may be driving mechanism.

The above measures may further involve providing an eye manipulation system, which may be integral part of the surgical robotic system or an add-on to extend the surgical robotic system with additional functionality. The eye manipulation system may comprise an eye gripping device mounted to an arm and an actuation subsystem to effect a movement of the eye gripping device. In this context, the term 'eye manipulation system' may refer collectively to the arm, the eye gripping device, and the actuation subsystem, which may together form the eye manipulation system. Therefore, the term 'eye manipulation system' may be used as a substitute for naming these individual components in subsequent references. The actuation subsystem which is configured to effect the actuation of the eye manipulation system may also be referred to as 'second' actuation subsystem while the actuation subsystem which is configured to effect the adjustment of the pose of the surgical instrument may also be referred to as 'first' actuation subsystem. Here, the terms 'first' and 'second' are merely used as identifiers and do not imply that the two actuation subsystems are separate. Namely, while they may be distinct, they may also be implemented by a single actuation subsystem or by actuators that are part of the same actuation subsystem.

The arm may be a mechanical arm, meaning that the arm may be actuated, for example directly by one or more actuators or by one or more actuators acting upon a component which moveably suspends the arm. The arm may have at least one degree of freedom which may allow the arm to assume different poses in accordance with the at least one degree of freedom. The eye gripping device may be mounted to a distal end of the arm. The term 'distal' may use a base of the arm as a point of reference, meaning that a distal section or end of the arm may be located away from the base and is typically nearest to the surgical target. The at least one degree of freedom of the arm may, mechanically and/or through actuator control, cause the arm to move the eye gripping device along a curved plane representing a curvature of the eyeball. The movement may involve both repositioning and reorienting the eye gripping device, which may allow the eye gripping device to follow the contour of the curved plane in both location and orientation. Through this movement along the contour of the curved plane, the eye gripping device may be guided along a surface of the eye.

The eye gripping device may be configured to engage the eyeball by exerting a grip on a fibrous surface layer of the eyeball. Such a fibrous surface layer may include the sclera and the cornea, which together provide a protective outer coating for the eye. By adjusting the pose of the arm when the eye gripping device is engaged with the eyeball, i.e., when the eye gripping device exerts a grip on the fibrous surface layer of the eyeball, the eyeball may be reoriented within the eye socket and with respect to the orbital cavity. By specifically moving the eye gripping device so that it follows the contour of the curved plane in both location and orientation, the forces exerted on the eye may be limited to those applied along the natural degrees of freedom of the eye, thereby avoiding or reducing the risk of damage that could occur if forces were applied in ways that the eye cannot naturally accommodate. In particular, the forces exerted by the eye gripping device on the eye may be mainly tangential to the surface instead of perpendicular, which may otherwise risk damaging the eye.

The inventors have considered that the use of an eye gripping device which grips a fibrous surface layer of the eyeball is advantageous over the use of surgical instruments to rotate the eyeball. Namely, surgical instruments may be challenging to manipulate due to the inherent risks associated with manipulating instruments inserted into the eye. Moreover, when using surgical instruments to rotate the eyeball, the forces may typically be applied to a trocar through the eye's surface, which may cause highly localized stress and potentially damage the surrounding tissue. To distribute these forces, multiple instruments may be used, but this may require coordinated manipulation, which is complex and may increase the risk of causing damage.

The eye gripping device may exert a grip on the fibrous surface layer, such as the sclera, which may be a protective and resilient layer of the eye. This grip may minimize the risk of severe damage since forces applied through the eye gripping device may be applied to an exterior surface rather than to an interior of the eye. Furthermore, the eye manipulator system may not require the insertion of surgical instruments into the eye, which may enhance its applicability at various stages of the procedure and reduce the risk of damage associated with inserted instruments.

Optionally, the surgical robotic system further comprises a processor subsystem configured to perform orbital manipulation of an eye by controlling the second actuation subsystem to move the eye gripping device when the eye gripping device is engaged with the eye. The eye manipulation system may be actuated under control of a computer, in that a processor subsystem may be provided to control the second actuation subsystem to actuate the arm. The processor subsystem may be configured, for example by a set of computer-readable instructions, to move the eye gripping device along the curved plane. This way, the eye manipulation system may autonomously or semi-autonomously, for example at the request of a surgeon, perform orbital manipulation. It is noted that the processor subsystem may be additionally configured to control the first actuation subsystem to control a position of the surgical instrument during the surgical procedure, for example relative to an intraocular surface during an intraocular procedure. In some embodiments, the processor subsystem may be configured to receive positioning instructions from a human operator, for example via a user input interface, and control the first actuation subsystem in accordance with the positioning instructions. Such type of control may also be referred to as master-slave or lead-follower control. In some embodiments, the processor subsystem may be configured to enable a human operator to exclusively control the position of the surgical instrument. In other embodiments, the processor subsystem may be configured to control the position of the surgical instrument semi-autonomously or even fully autonomously, for at least part of the surgical procedure. In such embodiments, there may be a possibility for the human operator to override an autonomous operation.

Optionally, the surgical instrument comprises an intraocular part for insertion into the eye via an entry point located on a surface of the eye, wherein the processor subsystem is configured to control the second actuation subsystem to perform orbital manipulation of the eye to reposition the entry point located on the surface of the eye with respect to the surgical instrument. The orbital manipulation may thus be performed for the purpose of adjusting the position of the entry point, for example relative to the surgical robotic system or a surgeon. This may facilitate access to the entry point, or may enhance the observability of a specific region within the eye, such as the anterior region. Alternatively, the processor subsystem may be configured to control the second actuation subsystem to perform orbital manipulation of the eye to primarily enhance the observability of a specific region within the eye.

Optionally, the surgical instrument has a variable remote center of motion which is repositionable along the surface of the eye, wherein the processor subsystem is configured to control the second actuation subsystem to perform orbital manipulation of the eye to align the entry point with the variable remote center of motion of the surgical instrument. During surgery, the variable remote center of motion (RCM) of the surgical instrument is typically positioned at the entry point into the eye, such as at a trocar inserted into the eye. The eye manipulation system may be used to perform orbital manipulation to rotate the eyeball to ensure that the entry point aligns with the variable RCM of the surgical instrument. This alignment may enable the surgical instrument to be inserted into the eye through longitudinal movement towards the eye.

Optionally, the processor subsystem is configured to control the first actuation subsystem and the second actuation subsystem to simultaneously reposition the variable remote center of motion and perform orbital manipulation to reposition the entry point. In cases where the actuation subsystems are separate, the processor subsystem may control distinct actuators to ensure that the RCM of the surgical instrument and the entry point of the eye remain co-located during orbital manipulation. This may allow for orbital manipulation while the surgical instrument is inserted into the eye. Conversely, if both actuation subsystems are integrated into a single subsystem, the processor subsystem may control this unified subsystem to simultaneously reposition the variable RCM and perform orbital manipulation to align the entry point.

Optionally, the eye gripping device is rotatable about a remote center of motion, wherein the remote center of motion of the eye gripping device is aligned with the variable remote center of motion of the surgical instrument, for example by mechanical alignment. By aligning the RCM of the eye gripping device with the RCM of the surgical instrument, either mechanically or through control of the actuation subsystem(s), the risk of forces being applied to the eye, for example by the inserted surgical instrument and/or by the eye gripping device, may be reduced. Such forces may otherwise potentially damage the eye or certain anatomical structures of the eye. Although these forces may also be mitigated or avoided by appropriately controlling the surgical instrument and the eye manipulation system to ensure that the RCM of the surgical instrument remains co-located with the entry point into the eye, the intrinsic alignment of RCMs may inherently reduce the risk and may obviate the need for complex synchronization in the control of both the surgical instrument and the eye manipulation system, or reduce the complexity of such synchronized control.

Optionally, the arm has multiple degrees of freedom, wherein the multiple degrees of freedom are aligned with axes of a coordinate system, for example a Cartesian or a polar coordinate system, wherein the processor subsystem is configured to control the second actuation subsystem to control the pose of the arm in the coordinate system to cause the eye gripping device to move along the curved plane. The arm may have a number of degrees of freedom which may enable the arm to be positioned in three-dimensional (3D) physical space. The processor subsystem may control the actuation subsystem to cause the arm to follow a specific trajectory within this 3D physical space to follow the curved plane. For example, the processor subsystem may control one or more actuators which directly act on the arm or control one or more actuators which act upon a component which moveably suspends the arm. This control may for example involve a coordinate conversion from coordinates defining the curved plane to a coordinate system which is used for the control of the arm. The computer control of the movement of the eye gripping device may be advantageous since computer control may improve the accuracy and flexibility of effecting the movement of the eye gripping device. For example, by way of the computer control, additional inputs may be taken into account to control aspects of the movement, such as user input, sensor input, a current stage of the procedure, etc.

Optionally, the processor subsystem is configured to use a geometrical model of the eye, for example a spherical or ellipsoid model, to internally represent the curved plane. The eye gripping device may be moved along the curved plane so that the eye gripping device follows the surface of the eye. By using a geometrical model of the eye, a geometric description of the surface of the eye may be obtained, which in turn may allow the processor subsystem to control the eye gripping device to follow this surface. Advantageously, by using an ellipsoid model, a more realistic model is used since the eye is typically ellipsoid in shape. This may allow the eye gripping device to more accurately follow the actual surface of the eye to ensure that forces are applied mainly tangential instead of perpendicular to the surface, reducing the risk of damage.

Optionally, the geometric model is a patient-specific model which is fitted to the eye of the patient. A patient-specific model may be a geometric model which is adapted, for example in terms of shape, to better match the actual eye geometry of a patient. By using a patient-specific model, the surface of the eye may be followed more accurately, further reducing the risk of damage to the eye when rotating the eye.

Optionally, the surgical robotic system further comprises a linkage system for movably suspending the instrument manipulator, wherein the linkage system is mounted to a base, wherein the arm is mounted to the instrument manipulator, to the linkage system, or to the base. By mounting the arm of the eye manipulation system to the instrument manipulator, to the linkage system, or to the base, the arm may be actuated by actuating one or more of these components of the surgical robotic system.

Optionally, the second actuation subsystem is arranged to actuate the arm by moving the base of the instrument manipulator. By moving a base to which both the arm of the eye manipulation system and the linkage system suspending the instrument manipulator are affixed, both the eye gripping device and the instrument manipulator may be manipulated. This may enable joint manipulation of the eye gripping device and the instrument manipulator, such as the coordinated movement of the RCM of the eye gripping device and the RCM of the instrument manipulator, by actuating a single base to which both components are movably suspended. This approach may reduce the complexity of control by eliminating the need to individually control separate actuation subsystems for the instrument manipulator and the eye gripping device.

Optionally, the arm has one or two degrees of freedom relative to the base, wherein the one or two degrees of freedom of the arm correspond to respective one or two rotational degrees of freedom of the eyeball within the eye socket. The eyeball may have two rotational degrees of freedom within the eye socket to enable horizontal rotation (yaw adjustment) and vertical rotation (pitch adjustment) within the eye socket. The arm may be unconstrained and unactuated in one of these rotational degrees of movement or in both of these rotational degrees of movement. Since the eye gripping device may be engaged with the eye, this configuration may enable the eye gripping device to be moved along the curved plane while its orientation towards the eye surface may be maintained due to the forces exerted by the grip. Consequently, it may not be necessary to separately actuate the arm nor the eye gripping device to have the eye gripping device to following the curved plane representing the eye surface.

Optionally, the arm is mechanically designed to limit movement of the eye gripping device to the curved plane. The arm may be mechanically designed and thereby mechanically constrained so that the degree(s) of freedom of the arm enable the eye gripping device to be moved along the curved plane while disallowing or at least limiting movement of the eye gripping device beyond the curved plane. This mechanical design may avoid the need for, or reduce the complexity of, software-based control, and may enable the arm to be operated in a relatively simple manner.

Optionally, the eye manipulation system further comprises a sensor configured to quantify the grip of the eye gripping device on the fibrous surface layer of the eyeball, for example to determine an efficacy of the grip or to determine a force exerted on the eye. By quantifying the grip of the eye gripping device on the fibrous surface layer, the eye manipulation system or another entity may act upon the quantification. For example, the quantification may allow assessment of whether the grip is sufficient, or whether the quality of the grip poses any risk of damaging the eye. The quantification of the grip may for example be used in a feedback loop to adjust the grip, for example by increasing a suction force onto the fibrous surface layer.

Optionally, the eye manipulation system further comprises a sensor data interface to access intraoperative pressure data acquired by an intraoperative pressure sensor, wherein the eye manipulation system is configured to use the intraoperative pressure data to verify that the eye manipulation system is operating within safe limits. Intraoperative pressure may be indicative of improper stress applied to the eye, which may potentially cause damage. By monitoring intraoperative pressure, issues with the grip on the eye may be identified and addressed. For example, if the intraoperative pressure exceeds a safety threshold, the grip on the eye may be released.

Optionally, the surgical robotic system further comprises a release mechanism to release the eye gripping device from the eye and/or to release the eye gripping device from the arm, for example in an emergency. In certain situations, such as in emergencies, it may be desirable to disconnect apparatuses and devices from the patient, for example to facilitate access to the patient or to allow the patient to be moved independently of such apparatuses and devices. Surgical instruments may be removed in an emergency, e.g., by hand or by the surgical robotic system which may be equipped with a release mechanism. The eye manipulation system may likewise be provided with a release mechanism to also allow the eye manipulation system to be disconnected from the patient. Such a release mechanism may release the eye gripping device from the eye and/or release the eye gripping device from the arm.

Optionally, the surgical robotic system further comprising a hold-and-release mechanism arranged for holding the instrument manipulator at a surgical position proximate to a patient and for releasing the instrument manipulator from the surgical position to a standby position distal to the patient, wherein the release mechanism is configured to effectuate the release of the eye gripping device when or before the hold-and-release mechanism releases the instrument manipulator from the surgical position to the standby position. By providing a hold-and-release mechanism, the surgical robotic system may be disconnected from the patient to allow for patient movement or access for emergency care. Releasing also the eye manipulation system from the patient may ensure a more complete disconnection. Releasing the eye manipulation system before or simultaneously with the instrument manipulator may be advantageous, particularly if the arm of the eye gripping device is mounted to a component that moves during the release, such as the instrument manipulator or a linkage system or the hold-and-release mechanism itself. This early or simultaneous release may help preventing forces generated during the disconnection from being transmitted through the eye manipulation system onto the eye, which may cause damage, for example when the eye gripping device is forcibly removed from the eye. Instead, the eye manipulation system may be proactively released from the eye in anticipation of or simultaneously with the surgical robotic system being disconnected.

Optionally, the release mechanism is configured to, when activated, release the eye gripping device from the eye by disengaging the grip on the fibrous surface layer of the eyeball. The eye manipulation system may be disconnected from the patient by the eye gripping device releasing its grip on the fibrous surface layer of the eyeball. For example, a suction force may be stopped to release the eyeball, the eyeball may be unclamped, a frictional contact may be ceased by withdrawing the contact, or needles may be withdrawn from the fibrous surface layer.

Optionally, the release mechanism is configured to, when activated, release the eye gripping device from the arm. The eye manipulation system may be disconnected from the patient by the eye gripping device being disconnected from the arm, for example by the arm releasing the eye gripping device or the eye gripping device releasing from the arm. The release mechanism may therefore not need to operate in close proximity to the eye, which may reduce the risk of accidental damage to the eye. When used in conjunction with the eye gripping device releasing its grip on the fibrous surface layer of the eyeball, this provides an additional safety measure.

Optionally, the eye gripping device is configured to exert the grip on the fibrous surface layer of the eyeball at multiple positions or across an area of the surface of the eyeball. By exerting the grip at multiple positions or across an area of the surface of the eyeball, the forces exerted by the eye gripping device may be spatially distributed over at least part of the fibrous surface layer, which may increase the efficacy of the exerted grip and may distribute stress across the fibrous surface layer, which in turn may reduce the localization of potentially damaging stress.

Optionally, the eye gripping device is configured to exert the grip on the fibrous surface layer of the eyeball in a releasable manner. The eye gripping device may be configured to be able to release the grip, for example without requiring a manual release, which may be advantageous, for example in emergency situations.

Optionally, the eye gripping device is configured to exert the grip on the fibrous surface layer of the eyeball by one or more of:
- creating a suction force on the fibrous surface layer of the eyeball;
- circumferentially clamping the eyeball;
- creating frictional contact between the fibrous surface layer of the eyeball and the eye gripping device;
- temporarily adhering or bonding the fibrous surface layer of the eyeball to the eye gripping device; and
- inserting needles into the fibrous surface layer of the eyeball.

The above-described techniques to exert the grip on the fibrous surface layer may each act on the protective and resilient layer of the eye, without requiring access to the interior of the eye. In particular, the needles may have a length and/or may be inserted in such a manner that individual needles do not penetrate completely through the fibrous surface layer or penetrate only through the fibrous surface layer but not through a next underlying layer, such as the underlying choroidal layer. In a specific example, respective needles may have an outer diameter of 600 µm or less, preferably 400 µm or less, more preferably 200 µm or less, and protrude by 600 µm or less, preferably 400 µm or less, from the contact plane. The above-described techniques may each be releasable. For example, a suction force may be stopped to release the eyeball, the eyeball may be unclamped, the frictional contact may be ceased by withdrawing the contact, the temporary adherence or bonding may be temporary in nature, and needles may be withdrawn from the fibrous surface layer.

Optionally, the arm has one or more degrees of freedom to enable the eye gripping device to be moved along a frontal axis in a nasal-temporal direction and/or along a longitudinal axis in a superior-inferior direction along the curved plane.

It will be appreciated by those skilled in the art that two or more of the above-mentioned embodiments, implementations, and/or aspects of the presently disclosed subject matter may be combined in any way deemed useful.

Modifications and variations of the eye manipulation system or the surgical robotic system, which correspond to the described modifications, variations, and optional aspects of another one of these entities and activities, may be carried out by a person skilled in the art on the basis of the present description.

### BRIEF DESCRIPTION OF THE DRAWINGS

These and other aspects of the presently disclosed subject matter are apparent from and will be elucidated with reference to the embodiments described hereinafter. In the drawings,
Fig. 1A schematically shows a field of view when viewing the eye straight ahead, which field of view encompasses the central posterior region of the eye;
Fig. 1B schematically shows a field of view obtained by rotating the eyeball within the eye socket, which field of view includes a peripheral posterior region of the eye;
Fig. 2 shows a sideways orientation of the eye during gonioscopy to, for example, visualize the trabecular meshwork using a gonio prism;
Fig. 3A schematically shows an eye gripping device which is mounted to an arm and which eye gripping device engages an eyeball by exerting a grip on the sclera;
Fig. 3B schematically shows an actuation of the arm to rotate the eyeball within the eye socket;
Figs. 4A-C each schematically show a different example of an arm and the actuation of the arm to rotate the eyeball within the eye socket;
Fig. 5 schematically shows a surgical robotic system comprising an instrument manipulator for holding a surgical instrument, wherein the surgical robotic system is configured to establish a variable remote center of motion of the surgical instrument;
Fig. 6A schematically shows the arm of the eye manipulation system mounted to the instrument manipulator of the surgical robotic system;
Fig. 6B schematically shows the arm of the eye manipulation system mounted to the hold-and-release mechanism of the surgical robotic system;
Fig. 6C schematically shows the arm of the eye manipulation system mounted to the base of the instrument manipulator of the surgical robotic system; and
Fig. 6D schematically shows the arm of the eye manipulation system mounted to a headrest for a patient.

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
- 100: eye
- 102: eyeball
- 104: eye socket
- 106: cornea
- 107: sclera
- 108: lens
- 110: field of view (central)
- 112: field of view (peripheral)
- 120: gonio prism
- 122: view axis
- 124, 125: eye rotation axis
- 130: eye gripping device
- 131: curved plane
- 132: arm
- 134: fork
- 135: joint
- 136: parallelogram mechanism
- 137, 138: hinge joint
- 140: movement
- 142: rotation
- 150, 151: allowed degree of movement
- 155, 156: axis of allowed degree of movement
- 160, 161: actuated degree of movement
- 165, 166: axis of actuated degree of movement
- 167: axes of actuated degree of movement
- 170: base
- 172, 174: actuated base
- 180: remote center of motion of eye gripping device
- 182: remote center of motion of surgical instrument
- 200: surgical robotic system
- 210: base
- 220: hold-and-release mechanism
- 230: (parallelogram) linkage system
- 240: instrument manipulator
- 250: surgical instrument
- 260: headrest

### DESCRIPTION OF EMBODIMENTS

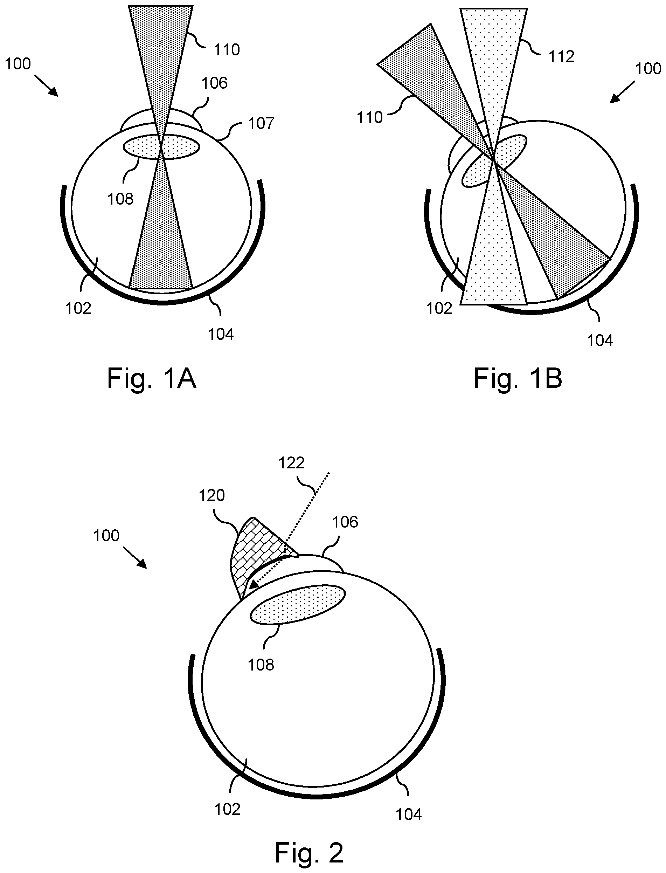

Figs. 1A, 1B, and 2 illustrate a need for orbital manipulation of the eye.

**Fig. 1A** shows various structures of the eye 100 including an eyeball 102, an eye socket 104, a cornea surface layer 106 (also referred to as 'cornea' in short), a sclera surface layer 107 (also referred to as 'sclera' in short), and a lens 108. Fig. 1A shows the eyeball 102 in a neutral orientation, looking substantially straight ahead and the eyeball 102 being substantially centrally aligned within the eye socket 104. To be able to see a posterior region of the eye, for example to obtain visual feedback during a surgery in the posterior region of the eye, an observer may wish to observe the posterior region through the cornea 106 and the lens 108, for example using an observation apparatus such as a microscope. Fig. 1A schematically shows the field of view 110 when the observer or observation apparatus views the eye 100 from a straight-ahead perspective. The field of view 110 is thereby limited to a central region of the posterior region of the eye 110 and does not reach far into the peripheral posterior region of the eye 110.

To address this deficiency, orbital manipulation may be performed, by which the eyeball 102 may be rotated within the eye socket 104. For example, a surgeon may rotate the eye using two instruments that are inserted into the eye (not shown in Fig. 1A). **Fig. 1B** shows a result of such orbital manipulation, showing the eyeball 102 having been rotated sideways within the eye socket 104. From the perspective of an observer or observation apparatus viewing the eye 100 from a straight-ahead perspective, the field of view 112, which is shown in a schematic manner in Fig. 1B, may now include a peripheral posterior region of the eye 100. For reference, the field of view 110 of the central posterior region of the eye 100 of Fig. 1A remains shown in Fig. 1B. The orbital manipulation may not only improve the visibility of the peripheral posterior region of the eye 100, but in some cases may also improve the reach of a surgical instrument and/or may allow certain types of tissue to be pushed within the field of view 112 by indentation of the sclera.

**Fig. 2** shows a sideways orientation of the eye 100 which may be obtained by orbital manipulation. At the sideways orientation of the eye 100, anatomical structures in the far periphery of the anterior chamber of the eye may be observed. For example, when observing the eye along a view axis 122 and when using a gonio prism 120, the trabecular meshwork may be observed during a gonioscopy procedure.

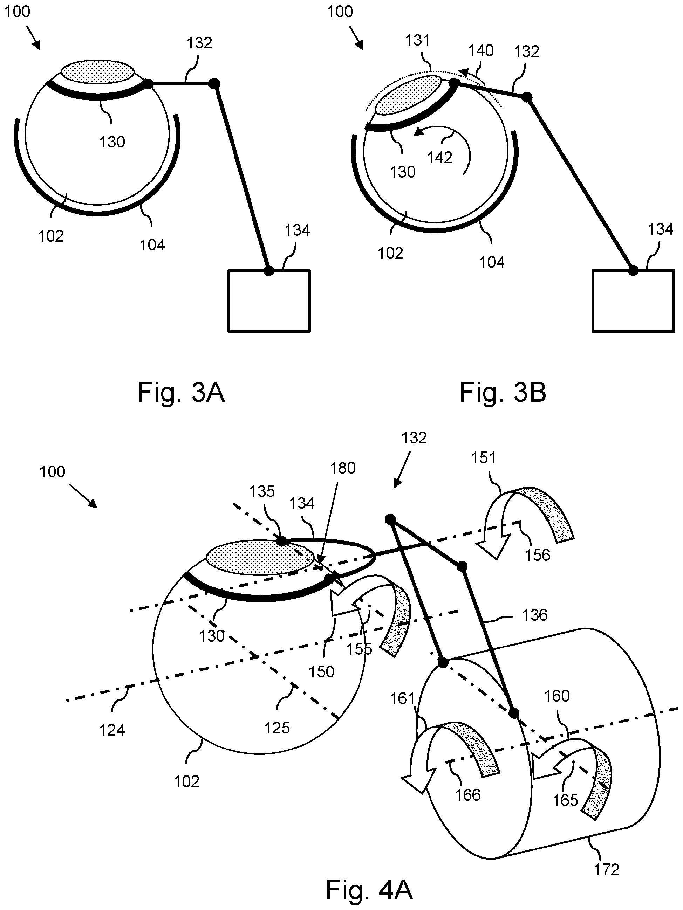

**Fig. 3A** shows an eye manipulation system for orbital manipulation of an eye of a patient. The eye manipulation system is shown to comprise an arm 132 having at least one degree of freedom and an eye gripping device 130 mounted to a distal end of the arm 132. The eye gripping device 130 may generally be configured to engage the eyeball 102 by exerting a grip on a fibrous surface layer of the eyeball. In the specific example of Fig. 3A and following, the eye gripping device 130 is shown as a ring-shaped device which engages the eyeball 102 by exerting a grip on the sclarea circumferentially around the cornea. For example, the ring-shaped device may be applied with a preload onto the sclarea to establish a friction-grip of the sclera and thereby of the eyeball 102. However, this is not a limitation, in that the eye gripping device 130 may engage the eyeball 102 in various other ways, for example by exerting the grip at different position(s) or in a different area of the fibrous surface layer, or by gripping the cornea or by gripping both the sclarea and the cornea. In general, the eye gripping device 130 may exert the grip on the fibrous surface layer of the eyeball in a releasable manner, for example by creating a suction force on the fibrous surface layer of the eyeball, by circumferentially clamping the eyeball, by creating frictional contact between the fibrous surface layer of the eyeball 102 and the eye gripping device 130, by temporarily adhering or bonding the fibrous surface layer of the eyeball 102 to the eye gripping device 130, and/or by inserting needles into the fibrous surface layer of the eyeball 102. Fig. 3A further shows the arm 132 to be connected to a base 170. Non-limiting examples of a base 170 will be described with reference to Figs. 4A-6D.

The arm 132 may be adjustable in pose to move the eye gripping device 130 along a curved plane representing a curvature of the eyeball 102. **Fig 3B** shows a result of such an adjustment in pose, which may for example be effected by actuating the arm 132, for example manually or using one or more actuators, to effect the movement of the eye gripping device 130 along the curved plane 131. Namely, by way of the movement 140 of the eye gripping device 130, the eyeball 102 may be rotated 142 within the eye socket 104. Such externally and purposefully induced rotation of the eyeball 102 within the eye socket 104 may also be referred to as orbital manipulation. It is noted that, for ease of interpretation, the curved plane 131 is shown in Fig. 3B significantly offset from the actual surface of the eye 100. In practice, the curved plane 131 may approximate and partially run through the actual surface of the eye 100. It is noted that actuation of the arm 132 may take place by one or more actuators directly acting upon the arm 132 and/or by one or more actuators acting upon the base 170.

The arm 132 may generally have a number of degrees of freedom which may allow the eye gripping device 130 to be moved along the curved plane 131 representing the surface of the eyeball 102. In another example, the arm 132 may have one degree of movement, for example only along the frontal axis in the nasal-temporal direction or only along the longitudinal axis in the superior-inferior direction.

In some examples, the eye manipulation system may comprise an actuation subsystem for actuating the arm and a processor subsystem configured to control the actuation subsystem to move the eye gripping device along the curved plane. For example, the actuation subsystem may comprise one or more actuators, and the processor subsystem may control the actuators accordingly. In a specific example, the actuators may comprise servo motors, stepper motors, or pneumatic cylinders, which provide controlled movement. The actuators may be controlled by the processor subsystem using various methods such as pulse-width modulation (PWM), digital signals, or analog control voltages. The processor subsystem may use feedback mechanisms such as encoders, potentiometers, or linear variable differential transformers (LVDTs) to monitor and adjust the position and speed of the actuators. Optionally, the processor subsystem may implement control strategies such as PID (Proportional-Integral-Derivative) control, feedforward control, or adaptive control.

In some examples, the arm may have multiple degrees of freedom, for example to allow the eye gripping device to be freely moved in a 3D volume within 3D physical space. The degrees of freedom of the arm may be aligned with axes of a coordinate system, for example a 3D Cartesian or a 3D polar coordinate system. In such examples, the processor subsystem may be configured to control the pose of the arm in the coordinate system to cause the eye gripping device to move along the curved plane. For control purposes, the processor subsystem may use a geometric model to internally represent the curved plane. For example, the geometric model may be a geometric model of the eye, for example a model of a surface or outline of the eye. In a specific example, the model of the eye may be a spherical model or an ellipsoid model to internally represent the curved plane. In another specific example, the model may be a patient-specific model, for example a parameterized spherical model or a parameterized ellipsoid model of which the parameters may have been previously fitted to measurement points indicative of an actual geometry of the eye of a patient. For example, a sphere may be parameterized by its center point (*x*₀,*y*₀,*z*₀) and its radius r, while an ellipsoid may be parameterized by its center point (*x*₀,*y*₀,*z*₀), semi-axes (*r*₁,*r*₂,*r*₃) and orientation (*α,β,γ*)*.* Techniques such as least squares fitting, optimization algorithms, or Hough transforms may be used to derive the parameters of a respective model from a set of measured surface points or from imaging data.

Alternatively to the arm having multiple degrees of freedom to allow the eye gripping device to be freely moved in a 3D volume within 3D physical space, the arm may be designed to mechanically limit movement of the eye gripping device to the curved plane. For example, the arm may be pivoting arm which may enable the eye gripping device to be moved along the curved plane while also limiting the movement of the eye gripping device beyond the curved plane. The pivoting arm may have length of the approximate radius of the eye. For example, the length may be approximately 12mm. The length of the pivoting arm may also be selected to correspond to the length of one of the semi-axes of the eye, e.g., a minor semi-axis or a major semi-axis.

Alternatively, or in addition to being actuated by one or more actuators, the arm may be manually actuatable. This may allow the user to move the eye gripping device along the curved plane by manually adjusting and thereby actuating the arm. For example, when the arm is mechanically designed to limit movement of the eye gripping device to the curved plane, such actuation may be easily effected by the user through simple physical actions such as pushing, pulling, or rotating of an arm part.

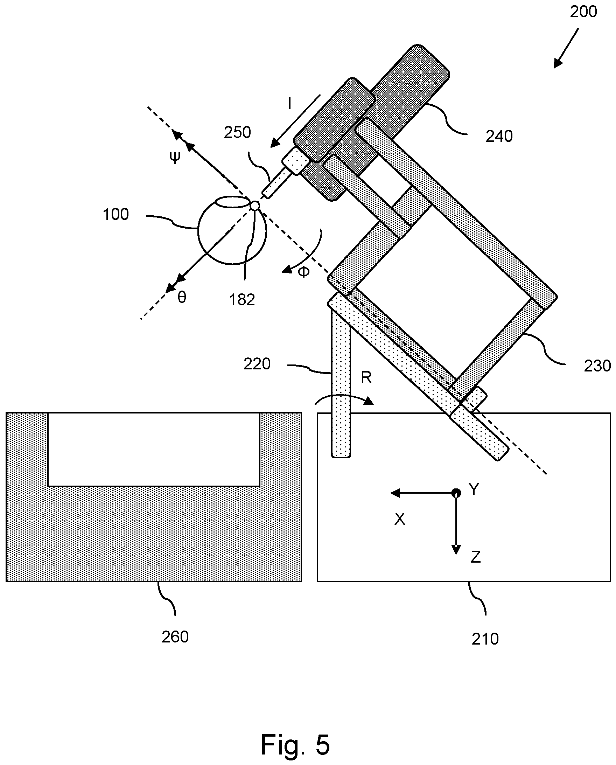

**Figs. 4A-4C** each schematically show an example of an arm 132 and its actuation to rotate the eyeball 102 of the eye 100 within the eye socket (eye socket not shown). The rotation of the eyeball 102 may be about a center of rotation which may be located at the intersection of axes 124, 125. Figs. 4A-4C further show an eye gripping device 130, being by way of example a ring-shaped eye gripping device 130. In each of Figs. 4A-4C, a proximate end of the arm 132 is mounted to an actuated base 172, 174. Through the design of the arm 132, the eye gripping device 130 at the distal end of the arm 132 is made to move along a curved plane by actuating the base 172, 174. Figs. 4A-4C each show a different exemplary design of the arm 132 and/or actuated base. As will be described with reference to Figs. 5-6D, the base may be a base of a surgical robotic system, and the actuation of the base may serve to jointly actuate an instrument manipulator and the eye manipulation system. However, it will be appreciated that instead of actuating the base of the arm 132, the arm itself may be actuated, e.g., by one or more actuators directly acting upon links of the arm 132. It will be further appreciated that the term `actuated arm' and similar terms and phrases include the arm 132 being actuated by a base, to which the arm is connected, being actuated.

**Fig. 4A** shows an example where the arm 132 comprises a parallelogram mechanism 136 which is attached to an actuated base 172. The actuated base may in some examples be an actuator, such as a motor, directly serving as base. In the example of Fig. 4A, the actuated base 172 may have two actuated degrees of movement (DoF) 160, 161 and corresponding DoF axes 165, 166. The parallelogram mechanism 136 may be attached to a fork 134, which fork 134 may be attached via joints 135 to the eye gripping device 130. The joints 135 may for example be ball joints providing three rotational DoFs or universal joints providing two rotational DoFs or hinge joints providing one rotational DoF. The fork 134 may provide the eye gripping device 130 with an allowed degree of movement 150 along the axis 155, while the parallelogram mechanism 135 may provide the eye gripping device 130 with an allowed degree of movement 151 along the axis 156. Here, the adjective 'allowed' may indicate that the eye gripping device 130 may be allowed to move along the respective DoF in the sense that the eye gripping device 130 is not otherwise prevented from such movement. Such an allowed DoF may elsewhere also be referred to as a 'released' DoF or an 'unconstrained' DoF. The terms 'allowed', 'released', and 'unconstrained' may therefore be used interchangeably. The parallelogram mechanism 135 and the fork 134 may effectively suspend the eye gripping device 130 to provide the eye gripping device 130 with two unconstrained DoF 150, 151. The intersection of axes 155, 156 may define a remote center of motion 180 of the eye gripping device 130. Through the actuation of the base 172, the eye gripping device 130 may be moved and due to its grip onto the eyeball 102 and its two unconstrained DoF 150, 151 may be made to follow the curvature of the eyeball 102.

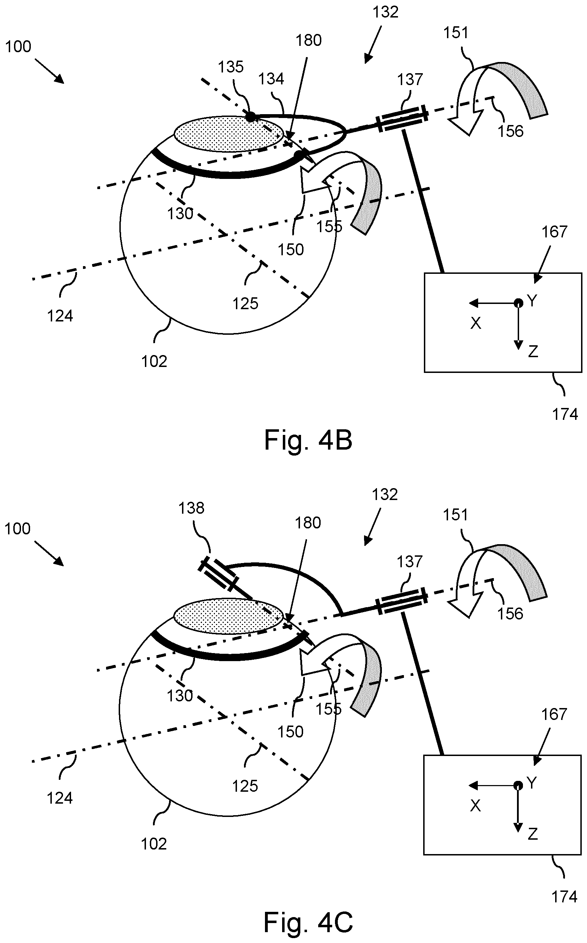

**Fig. 4B** shows an example where the arm 132 comprises a hinge joint 137 which connects the fork 134 to an actuated base 174, for example via one or more rods or linkages. As in the example of Fig. 4A, the fork 134 may be attached via joints 135 to the eye gripping device 130. The fork 134 may provide the eye gripping device 130 with an allowed degree of movement 150 along the axis 155, while the hinge joint 137 may provide the eye gripping device 130 with an allowed degree of movement 151 along the axis 156. The hinge joint 137 may therefore represent an alternative to the parallelogram mechanism 136 used in the example of Fig. 4A. In the example of Fig. 4B, the base 174 may be actuated to move in 3D physical space, for example along the three axes 167 of a XYZ Cartesian coordinate system or of a 3D polar coordinate system. Through the actuation of the base 174, e.g., in XYZ, the eye gripping device 130 may be moved and due to its grip onto the eyeball 102 and its two unconstrained DoF 150, 151 may be made to follow the curvature of the eyeball 102.

**Fig. 4C** shows another example where the arm 132 comprises a series configuration of a first hinge joint 137 and a second hinge joint 138 which together connect the actuated base 174 to the eye gripping device 130, for example via one or more rods or linkages. The arm 132 may therefore omit the fork 134 and its connecting joints 135 which were present in the Figs. 4A and 4B examples. Moreover, as in the example of Fig. 4B, the base 174 may be actuated to move in 3D physical space, for example along the three axes 167 of a XYZ Cartesian coordinate system or of a 3D polar coordinate system. Through the actuation of the base 174, e.g., in XYZ, the eye gripping device 130 may be moved and due to its grip onto the eyeball 102 and its two unconstrained DoF 150, 151 may be made to follow the curvature of the eyeball 102.

With continued reference to Figs. 4A-4C, it is noted that the arm 132 may be actuated manually by manually actuating the base. For example, a base which is movable in XYZ may be manually moved in 3D physical space so that the eye gripping device 130 may be moved and due to its grip onto the eyeball 102 and its two unconstrained DoF 150, 151 may be made to follow the curvature of the eyeball 102.

**Fig. 5** shows a surgical robotic system 200 which comprises an instrument manipulator 240 for mounting a surgical instrument 250. As such, the instrument manipulator 240 may also be referred to as a 'mount' for the surgical instrument 250. Another term for instrument manipulator may be `manipulator head'. The surgical instrument 250 may be a tool or device for performing specific actions of carrying out desired effects during a surgery or operation, such as modifying biological tissues or to provide access for viewing biological tissue. Examples of surgical instruments include, but are not limited to, forceps, mechanical cutters, coagulation cutters, scissors, injection needles, sealing devices, etc. The instrument manipulator 240 may be movably suspended by a linkage system 230. The linkage system 230 may for example be a parallelogram linkage system for positioning the instrument manipulator. In a specific example, the linkage system 230 may be configured to rotationally move the instrument manipulator from a surgical position proximate to a patient to a standby position distal to the patient. During the rotational movement, the instrument manipulator 240 may be suspended on a suspension point of the instrument manipulator via a linkage component coupled to the instrument manipulator. By way of the linkage system 230, or by movably suspending the instrument manipulator in another manner, the instrument manipulator 240 may be provided with least one degree of freedom to enable adjustment of a pose of the surgical instrument 250. For example, the surgical instrument 250 may be moved longitudinally in the direction I.

Fig. 5 further shows the surgical robotic system 200 to comprise a hold-and-release mechanism 220 arranged for holding the linkage system 230 and thereby holding the instrument manipulator 240 at a surgical position proximate to a patient and for releasing ('R' in Fig. 5) the linkage system 230 and thereby releasing the instrument manipulator 240 from the surgical position to a standby position distal to the patient. The hold-and-release mechanism 220 may be mounted to a base 210 of the surgical robotic system 200, which base 210 may be movable in 3D physical space, for example in a 3D Cartesian coordinate system, e.g., in X, Y, Z, or in a 3D polar coordinate system. Fig. 5 further shows a headrest 260 for holding a head of the patient. In some examples, the headrest 260 may be separate from the surgical robotic system 200 but may have a known physical position relative to the base 210 of the surgical robotic system 200. In other examples, the headrest 260 may be connected to the base 210 of the surgical robotic system 200, for example in a mechanical manner.

Although not shown in Fig. 5, the surgical robotic system 100 may comprise an actuation subsystem and a processor subsystem which may be jointly arranged to effect the adjustment of the pose of the surgical instrument 250. The actuation subsystem may comprise one or more actuators and the processor subsystem may control the actuator(s). For example, the actuation subsystem may actuate one or more of the base 210, the hold-and-release mechanism 220, the linkage system 230, and the instrument manipulator 240. In a specific example, the actuators may be of a type as previously described with reference to the actuation subsystem of the eye manipulation system. In a specific example, the processor subsystem may control the actuators using a control technique as previously described with reference to the processor subsystem of the eye manipulation system. Using the actuation subsystem, the surgical instrument 250 may be adjusted in pose to have a variable remote center of motion (RCM) 182 on a surface of the eye 100. For example, the surgical instrument 250 may be adjusted in pose in Z and Θ directions by actuation of the instrument manipulator 240, and in Φ direction and Ψ direction by actuation of the linkage system 230.

In some examples, the eye manipulation system as described in the present disclosure may be combined with a surgical robotic system as described in the present disclosure. For example, both systems may be mechanically aligned or connected, and may optionally share a processor subsystem which may jointly control respective actuation subsystems of the surgical robotic system and the eye manipulation system. The eye manipulation system may be part of the surgical robotic system, for example a subsystem thereof or an add-on extending functionality of the surgical robotic system.

In general, a surgical instrument, which may for example by held manually by a surgeon or by the aforementioned surgical robotic system, may comprise an intraocular part for insertion into the eye via an entry point located on a surface of the eye. The entry point may for example take the form of a trocar which is inserted through a surface of the eye, for example through the cornea. The eye manipulation system may be configured, for example by its processor subsystem being configured accordingly, to perform orbital manipulation of the eye to reposition the entry point located on the surface of the eye. In examples such as the Fig. 5 example where the surgical instrument 250 is held by a surgical robotic system 200 and in which the surgical instrument has a variable RCM 182 which is repositionable along the surface of the eye 100, the eye manipulation system may be configured to perform orbital manipulation of the eye 100 to align the entry point with the variable RCM 182 of the surgical instrument 250. For that purpose, the eye manipulation system may access data indicative of a repositioning of the RCM 182 of the surgical instrument 250. For example, if the eye manipulation system is part of the surgical robotic system 200, a processor subsystem may jointly manipulate the surgical instrument 250 and the arm to which the eye gripping device is attached (arm and eye gripping device not shown in Fig. 5). In a specific example, the processor subsystem may jointly control actuators of the eye manipulator system and the surgical robotic system to simultaneously reposition the variable RCM 182 and perform orbital manipulation of the eye 100 to reposition the entry point. If the eye manipulation system is an addon to the surgical robotic system, the eye manipulation system may be informed of the repositioning of the RCM 182, for example via a data interface to the surgical robotic system 200.

In another example, the eye manipulation system may be configured to perform orbital manipulation of the eye 100 for purposes other than aligning the entry point with the variable RCM 182 of the surgical instrument 250. For example, orbital manipulation may be performed to enhance the observation of a specific region within the eye, such as the anterior region. When the surgical instrument 250 is not inserted into the eye, the variable RCM 182 of the surgical instrument 250 may not need to be moved concurrently with the entry point, but may rather be moved independently of the orbital manipulation of the eye 100, for example before or after such orbital manipulation.

In some examples, the eye gripping device may be rotatable about an RCM (not explicitly shown in Fig. 5). For example, the arm of the eye manipulation system may be mechanically designed and/or controlled in such a manner that the eye gripping device rotates about the RCM. The RCM of the eye gripping device, which may generally either be a fixed of variable RCM, may be aligned with a longitudinal axis of the surgical instrument 250, for example by mechanical alignment or appropriate control of the actuation subsystem. Specifically, the RCM of the eye gripping device may be aligned with the RCM 180 of the surgical instrument, for example by mechanical alignment or appropriate control of the actuation subsystem.

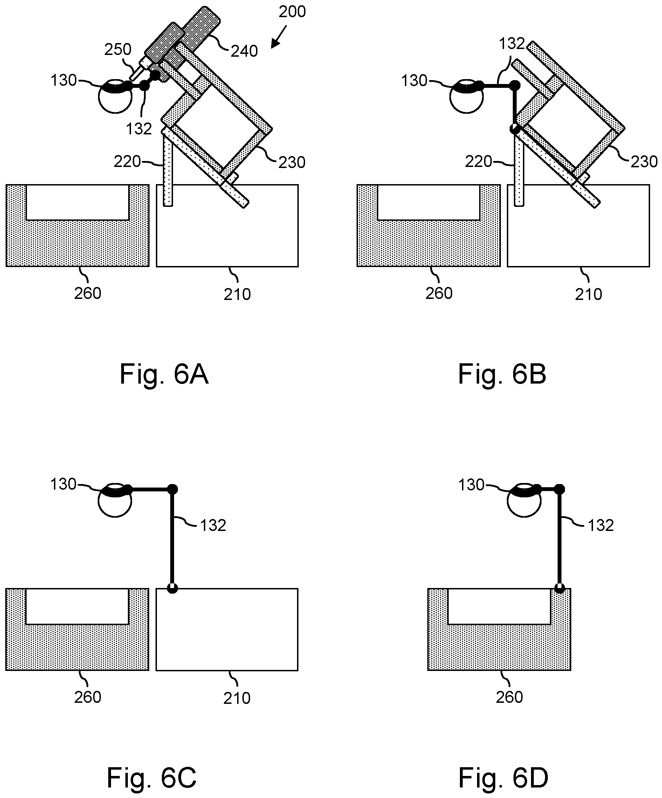

**Figs 6A-6D** illustrate various options for attaching the arm 132 of the eye manipulation system to the surgical robotic system 200 or to the headrest 260. Each attachment location may have its own specific degrees of freedom (DoF) that may be either constrained or unconstrained. An unconstrained DoF may also be referred to as a 'allowed' or `released' DoF. Therefore, the arm 132 may have different sets of constrained and/or unconstrained DoF for each attachment option. It is noted that Figs. 6A-6D show the arm 132 and attachment locations schematically and show different subsets of the components of the surgical robotic system 200 for ease of interpretation. Each attachment location may also be referred to as a 'base' for the arm 132. In Figs. 3A and 3B, this base for attachment is referenced by the reference numeral 170, while Figs. 4A-4C show actuated bases which are referenced by the reference numerals 172, 174.

**Fig. 6A** shows the arm 132 of the eye manipulation system mounted to the instrument manipulator 240 of the surgical robotic system 200. For example, the arm 132 may be mounted to a distal end of the instrument manipulator 240. The instrument manipulator 240 may be manipulated in Φ and Ψ direction. Here and in the following, the phrasing `may be manipulated' may refer to the respective entity having the corresponding DoF in which the entity is allowed to move and optionally actuatable to move. The various DoFs themselves, e.g., Φ, Ψ, X, Y, Z are visualized in Fig. 5. Since the instrument manipulator 240 may be moved in the Φ and Ψ directions, these DoFs may need to be released in the eye gripping device 130 to ensure that a gripped eye remains stationary in terms of position. Here, the term 'released' may refer to movement along a respective DoF being allowed, for example by the movement not being otherwise constrained. The arm 132 may be mounted to the instrument manipulator 240 in such a way that the RCM of the surgical instrument 250 and the RCM of the eye gripping device 130 each lie on a longitudinal axis of the surgical instrument 250, or specifically such that both RCMs substantially coincide, which may limit the risk of mutual misalignment. Both the arm 132 and the instrument manipulator 240 may be moved in X, Y, and/or Z direction (in short also 'XYZ') when the base 210 is moved in XYZ. The arm 132 may be designed in such a way that when the base 210 is moved in XYZ, the RCM of the eye gripping device may be repositioned to effect orbital manipulation of the eye. For example, the eye gripping device 130 may be movably suspended along the DoFs Φ and Ψ at the distal end of the arm 132, thereby releasing the DoFs Φ and Ψ. This release of the Φ and Ψ DoFs may allow for orbital manipulation of the eye when moving the arm 132 in XYZ since the eye will be held virtually at the RCM of the eye gripping device 130. Examples of such an arm 132 and actuated base have been previously described with reference to Figs. 4B and 4C.

The eye manipulation system may further comprise a release mechanism arranged to release the eye gripping device 130 from the eye and/or to release the arm 132 from the eye gripping device 130. The release mechanism may be arranged to operate synchronously with the hold-and-release mechanism 220 of the surgical robotic system 200 to ensure that when the instrument manipulator 240 is rotationally moved from the surgical position proximate to the patient to the standby position distal to the patient, for example in an emergency, that the connection of the arm 132 to the eye is also released. For example, the release mechanism may be configured to, when activated, release the eye gripping device 130 from the eye by disengaging the grip on the fibrous surface layer of the eyeball. Another example is that the release mechanism may be configured to, when activated, release the eye gripping device 130 from the arm 132, for example by disengaging a locking mechanism or deactivating a magnetic coupling. Such synchronous operation may, for example, be provided by a mechanical linkage system or through synchronized control of both release mechanisms.

**Fig. 6B** shows the arm 132 of the eye manipulation system mounted to the hold-and-release mechanism 220 of the surgical robotic system. The arm 132 may therefore not be subject to actuated movement of the instrument manipulator nor of the linkage system 230. When the base 210 is moved in XYZ, the arm 132 may be moved in XYZ. The arm 132 may be designed in such a way that when the base 210 is moved in XYZ, the RCM of the eye gripping device 130 may be repositioned to effect orbital manipulation of the eye. Examples of such an arm 132 and actuated base have been previously described with reference to Figs. 4B and 4C. In some examples, the arm 132 may be embodied as a 0-DoF fixed arm. In the example of Fig. 6B, the eye manipulation system may further comprise a release mechanism arranged to release the eye gripping device 130 from the eye and/or to release the arm 132 from the eye gripping device 130. The release mechanism may be of a type as previously discussed with reference to Fig. 6A.

Another example is that the arm 132 may be mounted to the linkage system 230. Since the linkage system 230 may be actuated to move along the Φ direction, the corresponding DoF may need to be released in the eye gripping device 130 to ensure that the gripped eye remains stationary when the linkage system is actuated. For example, the eye gripping device 130 may be movable suspended along the Φ DoF at a distal end of the arm 132. When the base 210 is moved in XYZ, the arm 132 may also be moved in XYZ. The arm 132 may be designed in such a way that when the base 210 is moved in XYZ, the RCM of the eye gripping device 130 may be repositioned to effect orbital manipulation of the eye. Examples of such an arm 132 and actuated base have been previously described with reference to Figs. 4B and 4C. The eye manipulation system may further comprise a release mechanism arranged to release the eye gripping device 130 from the eye and/or to release the arm 132 from the eye gripping device 130. The release mechanism may be of a type as previously discussed with reference to Fig. 6A. Fig. 6C shows the arm 132 of the eye manipulation system mounted to the base 210 of the surgical robotic system. The arm 132 may therefore not be subject to actuated movement of the instrument manipulator nor of the linkage system. When the base 210 is moved in XYZ, the arm 132 may be moved in XYZ. The arm 132 may be designed in such a way that when the base 210 is moved in XYZ, the RCM of the eye gripping device 130 may be repositioned to effect orbital manipulation of the eye. Examples of such an arm 132 and actuated base have been previously described with reference to Figs. 4B and 4C. The RCM of the eye gripping device 130 and the RCM of the surgical instrument may be mechanically fixed to each other when the release-and-hold mechanism holds the linkage system and instrument manipulator in the surgical position. Thereby, both RCMs may be aligned with each other. The release-and-hold mechanism may release the linkage system and instrument manipulator from the surgical position to the standby position without the eye manipulation system needing to release from the eye. When the linkage system and instrument manipulator again assume the surgical position, the RCM of the eye gripping device 130 and the RCM of the surgical instrument may be realigned with each other. The eye manipulation system may nevertheless comprise a release mechanism arranged to release the eye gripping device 130 from the eye and/or to release the arm 132 from the eye gripping device 130. The release mechanism may be of a type as previously discussed with reference to Fig. 6A.

**Fig. 6D** shows the arm 132 of the eye manipulation system mounted to the headrest 260 for the patient. The headrest 260 may lack any DoFs. As such, when the arm 132 of the eye manipulation system is attached to the headrest 260, the arm 132 may need to be actuated separately, e.g., manually or by an actuation subsystem of the eye manipulation system. For example, the Fig. 4A arm and actuator may be used in the example of Fig. 6D. The arm 132 may not be subject to actuated movement of the instrument manipulator, the linkage system nor of the base of the surgical robotic system. In this example, the release-and-hold mechanism of the surgical robotic system may release the linkage system and instrument manipulator from the surgical position to the standby position without the eye manipulation system needing to release from the eye. The eye manipulation system may nevertheless comprise a release mechanism arranged to release the eye gripping device 130 from the eye and/or to release the arm 132 from the eye gripping device 130. The release mechanism may be of a type as previously discussed with reference to Fig. 6A. In some examples, the arm 132 may be embodied as a 0-DoF fixed arm, that is, an arm lacking any DoFs.

Although not shown in the figures, the eye manipulation system may further comprise a sensor configured to quantify the grip of the eye gripping device on the fibrous surface layer of the eyeball, for example to determine an efficacy of the grip or to determine a force exerted on the eye. Examples of such sensors include, but are not limited to, strain gauges, force sensors, piezoelectric sensors, or optical sensors. The processor subsystem of the eye manipulation system may analyze the sensor data of the sensor to quantify the grip, and based on said quantification, take actions such as adjusting the grip of the eye gripping device, aborting a surgical procedure, limiting the movement of the eye gripping device, disconnecting the eye gripping device from the arm, etc.

The eye manipulation system may further comprise a sensor data interface to access intraoperative pressure data acquired by an intraoperative pressure sensor. The eye manipulation system may be configured to use the intraoperative pressure data to verify that the eye manipulation system is operating within safe limits. For example, when the intraoperative pressure data indicates that the intraoperative pressure exceeds a safety threshold, the release mechanism of the eye manipulation system may be activated so that the grip exerted on the eye may be released.

With continued reference to the eye gripping device, the following discusses some of the options for gripping the eye. For example, the eye gripping device may comprise an eye cup that can be placed on the surface of the eye. The eye cup may exert a grip on the eye's surface by creating a vacuum beneath the cup. An eye cup may also be referred to as a vacuum cup. Another example is a ring that may be placed onto the surface of the eye. The ring may be applied with a preload to generate a sufficient yet minimal amount of friction to exert a grip on the surface of the eye. Yet another example is that the eye gripping device may comprise a number of needles which may be inserted into the fibrous surface layer and thereby into the external ocular tissue. The needles may for example be arranged as one or more needle beds. The depth of the needles into the tissue may be shallow, e.g., not penetrating completely through the fibrous surface layer, and the diameter may be small, for example 29G (approximately 320 µm) or less, preferably 33G (approximately 200 µm) or less, more preferably 36G (approximately 160 µm) or less. The needles may, for example, be placed orthogonally with respect to the sclera or may be placed radially into the cornea, near the corneal limbus. Another example is that the needles may be placed in the most anterior section of the sclera, next to the corneal limbus part of the sclera radially. The needles may also have different relative orientations with respect to the fibrous surface layer, for example with groups of needles having an opposing direction with respect to the surface normal of the eye, for example forming a V-shaped configuration. In general, the eye gripping device may be configured to exert the grip on the fibrous surface layer of the eyeball at multiple positions or across an area of the surface of the eyeball. In general, the eye gripping device may be placed onto the conjunctiva but may be designed to exert the grip in such a manner that the grip, for example by means of a force, may be effectively transferred to the sclera. In general, the surface with which the eye gripping device engages the eye may have different shapes, for example ring-shaped. Another example is that the engaging surface may be formed by one or multiple arcuate segments but which segment(s) do not form a complete ring. Yet another example is that the engaging surface may be formed by one or more surface patches.

In a specific yet non-limiting embodiment, the eye gripping device may comprise an eye cup, the arm may be mounted to the base of the surgical robotic system, and the eye cup may be suspended in 2 DoFs relative to the arm, for example for posterior surgery, and in 1 DoF for anterior surgery. In such an embodiment, the release mechanism may be configured to detach the eye gripping device from the arm, for example in case of an emergency. In another specific yet non-limiting embodiment, the eye gripping device may comprise an eye cup, the arm may be mounted to the linkage system or to the hold-and-release mechanism, the eye cup may be suspended in 2 DoFs or in 1 DoF relative to the arm. In such an embodiment, the release mechanism may be configured to detach the eye gripping device from the eye.

It should be noted that the above-mentioned embodiments illustrate rather than limit the presently disclosed subject matter, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The mere fact that certain measures are recited in mutually different dependent claims does not indicate that a combination of these measures cannot be used to advantage.

## Claims

1. A surgical robotic system (200) for ophthalmic surgery, comprising:
- an instrument manipulator (240) for mounting a surgical instrument (250), the instrument manipulator having at least one degree of freedom to enable adjustment of a pose of the surgical instrument;
- a first actuation subsystem arranged to effect the adjustment of the pose of the surgical instrument;
- an arm (132) having at least one degree of freedom;
- an eye gripping device (130) mounted to a distal end of the arm, wherein the eye gripping device is configured to engage an eyeball (102) by exerting a grip on a fibrous surface layer of the eyeball;
- a second actuation subsystem arranged to effect a movement of the eye gripping device along a curved plane (131) representing a curvature of the eyeball to, when the eyeball is engaged, rotate the eyeball within an eye socket (104).

2. The surgical robotic system (200) according to claim 1, further comprising a processor subsystem configured to perform orbital manipulation of an eye (100) by controlling the second actuation subsystem to move the eye gripping device (130) when the eye gripping device is engaged with the eye.

3. The surgical robotic system (200) according to claim 2, wherein the surgical instrument (250) comprises an intraocular part for insertion into the eye (100) via an entry point located on a surface of the eye, wherein the processor subsystem is configured to control the second actuation subsystem to perform orbital manipulation of the eye to reposition the entry point located on the surface of the eye with respect to the surgical instrument.

4. The surgical robotic system (200) according to claim 3, wherein the surgical instrument (250) has a variable remote center of motion (182) which is repositionable along the surface of the eye (100), wherein the processor subsystem is configured to control the second actuation subsystem to perform orbital manipulation of the eye to align the entry point with the variable remote center of motion of the surgical instrument.

5. The surgical robotic system (200) according to claim 4, wherein the processor subsystem is configured to control the first actuation subsystem and the second actuation subsystem to simultaneously reposition the variable remote center of motion (182) and perform orbital manipulation to reposition the entry point.

6. The surgical robotic system (200) according to claim 4 or 5, wherein the eye gripping device (130) is rotatable about a remote center of motion (180), wherein the remote center of motion of the eye gripping device is aligned with the variable remote center of motion (182) of the surgical instrument (250), for example by mechanical alignment.

7. The surgical robotic system (200) according to any one of claims 1 to 6, wherein the arm (132) has multiple degrees of freedom, wherein the multiple degrees of freedom are aligned with axes of a coordinate system, for example a Cartesian or a polar coordinate system, wherein the processor subsystem is configured to control the second actuation subsystem to control a pose of the arm in the coordinate system to cause the eye gripping device (130) to move along the curved plane (131).

8. The surgical robotic system (200) according to any one of claims 1 to 7, wherein the arm (132) has one or more degrees of freedom to enable the eye gripping device (130) to be moved along a frontal axis in a nasal-temporal direction and/or along a longitudinal axis in a superior-inferior direction along the curved plane (131).

9. The surgical robotic system (200) according to any one of claims 1 to 8, further comprising a linkage system (230) for movably suspending the instrument manipulator, wherein the linkage system is mounted to a base (210), wherein the arm (132) is mounted to the instrument manipulator, to the linkage system, or to the base.

10. The surgical robotic system (200) according to claim 9, wherein the second actuation subsystem is arranged to actuate the arm (132) by moving the base (210) of the instrument manipulator (240).

11. The surgical robotic system (200) according to claim 10, wherein the arm (132) has one or two degrees of freedom relative to the base (210), wherein the one or two degrees of freedom of the arm correspond to respective one or two rotational degrees of freedom of the eyeball (102) within the eye socket (104).

12. The surgical robotic system (200) according to any one of claims 1 to 11, further comprising a release mechanism arranged to release the eye gripping device (130) from the eye (100) and/or to release the eye gripping device from the arm.

13. The surgical robotic system (200) according to claim 12, further comprising a hold-and-release mechanism (220) arranged for holding the instrument manipulator (240) at a surgical position proximate to a patient and for releasing the instrument manipulator from the surgical position to a standby position distal to the patient, wherein the release mechanism is configured to effectuate the release of the eye gripping device (130) when or before the hold-and-release mechanism releases the instrument manipulator from the surgical position to the standby position.

14. The surgical robotic system (200) according to any one of claims 1 to 13, wherein the eye gripping device (130) is configured to exert the grip on the fibrous surface layer of the eyeball (102) at multiple positions or across an area of the surface of the eyeball.

15. The surgical robotic system (200) according to any one of claims 1 to 14, wherein the eye gripping device (130) is configured to exert the grip on the fibrous surface layer of the eyeball (102) in a releasable manner, for example by one or more of:
- creating a suction force on the fibrous surface layer of the eyeball;
- circumferentially clamping the eyeball;
- creating frictional contact between the fibrous surface layer of the eyeball and the eye gripping device;
- temporarily adhering or bonding the fibrous surface layer of the eyeball to the eye gripping device; and
- inserting needles into the fibrous surface layer of the eyeball.
